(19) Europäisches Patentamt
European Patent Office
Office européen des brevets

(11) Publication number: **0 381 529 A2**

(12) # EUROPEAN PATENT APPLICATION

(21) Application number: **90301140.1**

(51) Int. Cl.[5]: **A61L 9/12**

(22) Date of filing: **02.02.90**

(30) Priority: **02.02.89 US 305262**

(43) Date of publication of application: **08.08.90 Bulletin 90/32**

(84) Designated Contracting States: **DE FR GB NL**

(71) Applicant: **HERCULES INCORPORATED**
**Hercules Plaza**
**Wilmington Delaware 19894(US)**

(72) Inventor: **Gebbia, Barbara C.**
**4 Dey Hill Trail, Passaic County**
**Totowa, New Jersey 07512(US)**

(74) Representative: **De Minvielle-Devaux, Ian Benedict Peter et al**
**CARPMAELS & RANSFORD 43, Bloomsbury Square**
**London WC1A 2RA(GB)**

(54) **Air freshener.**

(57) An air freshening device comprising a carrier, characterized in that said carrier supports at least one encapsulated fragrance matrix, said encapsulated fragrance matrix comprising fragrance and encapsulation material, said encapsulation material being solvent soluble, said fragrance being encapsulated by said encapsulation material, said encapsulation matrix being supported by said carrier, said carrier being substantially insoluble in said solvent.

EP 0 381 529 A2

# AIR FRESHENER

The invention relates to air freshener carriers and wick air fresheners using air freshener carriers. In particular, the invention relates to refillable air fresheners in which a liquid is elevated by capillarity through a wick to a carrier which supports encapsulated fragrance.

Brenner et al., U.S. Patent 3,971,852, the disclosure of which is incorporated herein by reference in its entirety, disclose a process of microencapsulating an oil. Mettler, et al., in U.S. Patent 4,301,095; Spector, in U.S. Patent 4,346,059 and Mason, in U.S. Patent 4,268,285, disclose air fresheners which include a wick or wicking material. Baer, in U.S. Patent 4,572,375, discloses a container for dispersant material which includes a base member and a removable cover which fits over the base member. The base member includes a reservoir mounting collar for holding a reservoir of dispersant material in place. In U.S. Patent No. 3,727,840, a wick material is held in place in a bottom hemispherical shell and a dispersant is enclosed in a top hemispherical shell by a puncturable membrane. When the dispersant is used up, the entire container is disposed of.

There is a need for a wick air-freshener which is adapted to provide initial rapid release of encapsulated fragrance from a readily replaceable carrier as is provided by the present invention.

Accordingly, the invention provides an air freshener comprising a sheet, characterized in that said sheet comprises a carrier and at least one encapsulated fragrance matrix, said encapsulated fragrance matrix comprising fragrance and solvent soluble encapsulation material, said fragrance being encapsulated by said encapsulation material, said encapsulation material being supported by said carrier, said carrier being substantially insoluble in said solvent, said carrier being adapted to propagate said solvent therethrough by capillarity.

Also provided in accordance with the invention is an air freshener comprising a container, a wick, and a replaceable fragrance carrier, characterized in that said carrier comprises encapsulated fragrance, said encapsulated fragrance comprising a fragrance and a solvent soluble encapsulation material.

According to the invention, an air freshener system is provided, which uses a replaceable carrier having the improvement wherein the carrier contains encapsulated fragrance. The air freshener system includes a container, a wick and a carrier. Liquid solvent is added to the container. The wick elevates the solvent from the container to the carrier. The fragrance in the carrier is encapsulated in solvent soluble material. The solvent elevated to the carrier readily dissolves the encapsulation material and releases the fragrance into the ambient air. The carrier is readily replaceable.

The advantages provided by the invention include: i) a wick air-freshener which is adapted to provide initial rapid release of fragrance into the ambient air, ii) a readily replaceable carrier which supports encapsulated fragrance having solvent soluble encapsulation material, iii) a readily replaceable carrier which supports encapsulated fragrance and is adapted to propagate a nonflammable liquid therethrough by capillarity, and iv) a system and process to transfer liquid solvent from a container through a wick to a readily replaceable carrier which supports fragrance encapsulated within readily solvent soluble encapsulation material.

Figure 1 shows an exploded side view of an air freshener system, in accordance with the present invention.

Figure 2 shows an assembled side view of an air freshener in operative position in accordance with the invention.

The invention is now described with more particular reference to the Figures 1 and 2. In Figure 1, an air freshener system 10, in accordance with the invention, is shown. The air freshener system 10 includes a carrier 12, wick 14, refillable container 16, and cap 18. The container 16 encloses water (a liquid solvent) 20. Container 16 is refillable and reusable. The carrier 12 may be a water absorbant paper blotter. The carrier 12 preferably includes sheets of cellulosic paper 22 and 24. However, other shapes and other water absorbent material may be used. The cellulosic paper (or other water absorbent material) is impregnated with encapsulated fragrance 26. The encapsulated fragrance 26 includes a fragrance and a water soluble encapsulation material. The carrier 12 is supported by the cap 18. The cap 18 is supported by container 16.

Capillarity is the attraction between molecules, which results in the rise of a liquid in small tubes, fibers (such as in wick 14) or in the wetting of a solid (such as carrier 12) by a liquid (such as water 20). In operation, the lower end of the wick 14 is in contact with water 20, and the upper end of the wick 14 is in contact with carrier 12. The wick 14 draws water from container 16 to carrier 12. The encapsulation material is water soluble. The contact of the water with the encapsulated fragrance in the carrier 12 releases the fragrance from its encapsulating material. The fragrance then evaporates into the air surrounding the carrier 12. The capillary action of the water in the wick quickly draws the water from the container 16 through the wick 14 to

carrier 12. The water dissolves the water soluble material encapsulating the fragrance. The fragrance then evaporates into the atmosphere surrounding the carrier. When the fragrance is depleted from the carrier 12, the depleted carrier 12 is replaced by a replacement carrier containing a substantial proportion of encapsulated fragrance. The replacement carrier may be of the same or a different shape from carrier 12. Carriers may be any shape, such as disk, spherical, cylindrical or ornamental, for example, having the shape of a flower. Water is added to the container 16, as needed, to replace water which has evaporated and/or travelled up the wick.

Figure 2 shows an exploded view of the air freshener system 10. During storage, while fragrance dispensing is not required, the carrier 12 may be lifted from contact with the wick 14. Alternatively, water 20 and/or wick 14 may be removed from container. An unused carrier is placed in contact with wick 14 when air freshening is to be started for the most effective dispensing of fragrance.

To add water to container 16, cap 18 and wick 14 are lifted from the container 16. Water is then poured into the opening in the top of the container. The wick 14 is then placed into the water 20, and cap 18 is then placed on container 16. Carrier 12 contacts and supports wick 14 as cap 18 is placed onto container 16.

Different encapsulation materials may be used to vary the rate of release of fragrance. By combining fragrance encapsulated in several such materials in one carrier a continuous release of fragrance is provided. The fragrance may be microencapsulated or macroencapsulated.

The encapsulated fragrance 26 provided in the carrier 12 is encapsulated using water soluble encapsulation materials which preferably are combinations of matrix forming materials. These matrix forming encapsulation materials are characterized by the ability to form a continuous phase of an emulsion in which a high proportion of oil can be held in the dispersed phase. These preferred encapsulation materials are also characterized by plasticity or flowability in the drying temperature range during which a solid is derived from the emulsion by removal of moisture, e.g. when the emulsion is converted into particles by a spray drying procedure. The particles or capsules obtained by spray drying such an emulsion are largely spherical without substantial oil escape paths in the wall formed from the dissolved combination of matrix forming solids in the continuous phase of the emulsion due to discontinuities such as craters or pits, cracks, fissures, spin holes and the like. The result is a high oil recovery and low extractable oil content.

The practical upper limit of oil in the solid matrix is obtained by properly removing moisture from an emulsion having the maximum oil content that can be maintained in the dispersed phase. This oil content varies somewhat in different combinations of the matrix forming material but the maximum content for any particular combination is easily determined by observation of the mixture which undergoes a phase inversion when the oil content thereof is increased above this maximum limit.

The matrix resulting from removal of moisture from the emulsion, whether in the form of a sheet, block or particles, appears to have a glassy, amorphous cellular structure characteristic of materials that remain in liquid phase during moisture removal and solidify without substantial subsequent shrinkage. The body of the matrix in section is composed of tiny globules of oil that may be about one micron in diameter, although other diameters are obtainable by varying the technique of forming the emulsion.

One combination of matrix forming materials preferred for use as the encapsulation material comprises mixtures of polysaccharides and polyhydroxy compounds which form emulsions with the oil. These emulsions have a plastic or flowable state over a substantial range of temperature that is in a critical range over which water is readily removed between the fully liquid and fully solid states. They also form a surface that selectively permits removal of water and they become, on removal of moisture, a cellular matrix of the polysaccharide and polyhydroxy materials in solid state with oil fixed in the cells thereof. Materials other than polysaccharides and polyhydroxy compounds that, in combination, satisfy these criteria may be substituted for part or all of one or both of these materials.

The polysaccharides employed in admixture with polyhydroxy compounds to encapsulate fragrance are solids characterized by solubility in water and by at least partial solubility in, or capability of at least partially dissolving, the polyhydroxy compound with the ranges of proportions used. They are primarily not the sweet, readily soluble saccharides like sugar but higher polysaccharides that may be natural, such as gum arabic and similar vegetable gums, or synthetic, such as degradation and modified products of starch, which usually form colloidal solutions. Certain starch degradation products such as dextrinized starch which are suitable polysaccharides for use in system and process of the invention contain a wide spectrum of saccharides of different molecular weights including a sufficient proportion in the polysaccharide molecular weight range to be good encapsulants and varying proportions of lower saccharides such

as mono-, di- and trisaccharides which are polyhydroxy compounds, as later defined. When such polysaccharides are used it may be necessary to make adjustments in the proportion of added polyhydroxy compound in order to obtain the proper balance of polysaccharide and polyhydroxy compounds to assure proper melt characteristics as later described since the proportion of lower saccharides in the starch degradation products, while usually too low to satisfy the requirements of the invention for polyhydroxy compound, may be large enough to affect significantly the amount of added polyhydroxy compound required to obtain the said proper proportion of polyhydroxy compound to polysaccharide in the product.

Among the polysaccharides that may be used are dextrins derived from ungelatinized starch acid esters of substituted dicarboxylic acids represented diagrammatically by the formula:

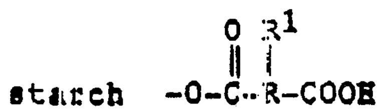

in which R is a radical selected from the class consisting of dimethylene and trimethylene and $R^1$ is a hydrocarbon substituent of R selected from the class consisting of alkeyl, alkenyl, aralkyl and aralkenyl groups. these ungelatinized starch-acid esters are prepared by reacting an ungelatined starch, in an alkaline medium, with a substituted cyclic dicarboxylic acid anhydride having the following formula:

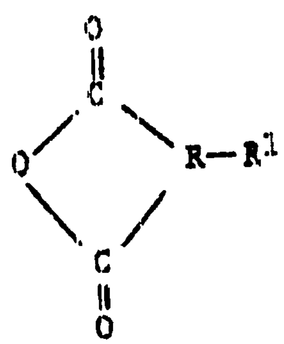

in which R and $R^1$ represent the so designated substituent groups just defined. Examples of such anhydrides are the substituted succinic and glutaric acid anhydrides. Other useful polysaccharides include products derived from dextrinized starch, and hydrolyzed starch. In general, these products contain minor proportions of lower saccharides such as dextrose. The polysaccharide content may comprise a single polysaccharide or mixture of two or more polysaccharides.

The polysaccharide should possess emulsifying properties either inherently or by reason of the presence of a minor proportion of a suitable emulsifying agent. Further definition of emulsifying agents is unnecessary because they are well known to those skilled in the art. Examples of satisfactory emulsifying agents are sodium diisoctyl sulfosuccinate and sodium caseinate. If emulsifying agents are added, proportions in the range of 0.1 to 10% based on the weight of polysaccharide in the mixture are satisfactory. An important property of the polysaccharide or polysaccharide-emulsifier combination is that when dissolved in water with the polyhydroxy compound the aqueous phase (a) is capable of emulsifying oil to form the dispersed phase of an oil-in-water emulsion with the oil globules having diameters largely within but not limited to the range of about 0.5 to 5 micron and (b) has sufficient stability not to invert or coalesce prior to moisture removal, e.g., by spray drying.

The polyhydroxy compounds employed in admixture with polysaccharide material as the encapsulation material for encapsulated fragrance 12. Such compounds are characterized by solubility in water and at least partial solubility in the polysaccharide material or capability of at least partially dissolving such material. Polyhydroxy compounds form with the polysaccharide material into a liquid melt which softens within the range of from 30 to 100° F for the ranges of proportions used. With the polysaccharide material the polyhydroxy compounds form a continuous aqueous phase in which oil is dispersible as a discontinuous phase to form a stable emulsion. The plasticity of the surface of the particles formed from the emulsion diminishes as water is removed through a drying operation, and the particles formed are in a solid state at the temperature of use.

Useful polyhydroxy compounds can be classified in these groups: alcohols, sugars from plant sources and those containing other functional groups. Polyhydroxy alcohols include glycerine, sorbitol, mannitol, erythritol and ribitol. The sugars from plant sources, include monosaccharides such as glucose, disaccharides such as maltose and sucrose, trisaccharides such as raffinose, and ketosaccharides such as fructose. These will be referred to as plant-type sugars whether actually derived from plants or produced synthetically. Polyhydroxy compounds containing other functional groups include glucuronolactone (lactone), sorbitan and mannitan (monoethers) and methylglucopyranoside (acetal).

In general, the proportion of polyhydroxy compounds is at least 20% by volume of the encapsulated fragrance matrix. The suitability of mixtures of these matrix forming materials, e.g., polysaccharide material and polyhydroxy compounds for use in the present invention may be determined by the solubility procedures set forth in U.S. Patent 3,971,852 and Softening Temperature Range Test.

The fragrance oils that can be encapsulated in accordance with the present invention include nonvolatile as well as volatile oils using spray drying. The oils are characterized by being insoluble but dispersible (emulsifiable) in water and they may be volatile or nonvolatile under drying conditions which include elevated temperature and low relative humidity in the air stream. They are usually liquid at the temperature of the emulsion but petroleum jelly can be successfully encapsulated for use in the process of the invention since it is readily broken up into tiny particles in an emulsifying machine producing high shear. Among the volatile oils that can be encapsulated effectively for use in the present invention are natural and synthetic essential oils or compounded fragrance oils such as citrus (orange, lemon, lime and the like), spice oils (cascia, clove, wintergreen and the like), mint oils, (spearmint, peppermint, and the like), woody oils (vetiver, patchouli, and the like); perfume oils and individual components thereof, such as linalool, methyl salicylate, limonene, menthol, decanol, diethyl phthalate, carvone, citral, and the like.

The proportions of fragrance oil to encapsulate in the encapsulated fragrance matrix may vary widely from small but effective amounts to as high as 80% by volume of the encapsulated fragrance matrix. Preferably, the fragrance amounts to from 5 to 80 percent by volume of the encapsulated fragrance matrix. The principal benefits of the present invention in high yield and low extractable oil are realized in greatest measure where the fragrance oil amounts to at least 30% by volume of the encapsulated fragrance matrix.

## Claims

1. An air freshening device characterized by the combination of a carrier and a fragrance which is in the form of an encapsulated fragrance matrix, comprising fragrance and encapsulation material, the fragrance being encapsulated by the encapsulation material, the encapsulation material being solvent soluble and the carrier being substantially insoluble in said solvent.

2. The device of Claim 1 characterized in that it comprises a wick, which is positioned in contact with the carrier and which is adapted to transfer solvent by capillarity to the carrier.

3. The device of Claim 2 characterized in that it comprises a container which supports the wick.

4. The device of any of Claims 1-3 wherein the fragrance comprises a natural essential oil, a synthetic essential oil, compounded fragrance oil, perfume oil or individual components thereof.

5. The device of any of Claims 1-4 wherein the fragrance forms 5 to 80% by volume of the encapsulated fragrance matrix.

6. The device of any of Claims 1-5 wherein the solvent is water.

7. The device of Claim 6 wherein the encapsulated fragrance matrix is formed by encapsulating a fragrance oil in a solid encapsulation material, the said material comprising a mixture of a modified starch derived from ungelatinized starch acid esters of substituted dicarboxylic acids represented by the formula:

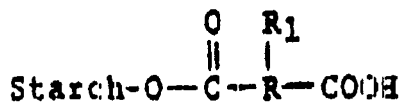

in which R is dimethylene or trimethylene and $R_1$ is a hydrocarbon selected from alkyl, alkenyl, aralkyl and aralkenyl groups, and a polyhydroxy compound present in an amount at least 20% by weight of the mixture and selected from alcohols, plant-type sugars, lactones, monoethers and acetals.

8. The device of Claim 7 which is the spray dried product of an emulsion of the fragrance oil in a solution of the modified starch and polyhydroxy compound in water.

9. The device of Claim 8 wherein the modified starch is a hydrolyzed starch.

10. The device of any of Claims 1-9 wherein the carrier is in sheet form.

11. The device of any of Claims 1-10 wherein the carrier comprises paper.

12. The device of any of Claims 1-11 wherein the carrier is adapted to propagate the solvent therethrough by capillarity.

13. An air freshener comprising a container, a wick, and a replaceable fragrance carrier, characterized in that the carrier supports an encapsulated fragrance matrix, the encapsulated fragrance matrix comprising a fragrance and a solvent soluble encapsulation material.

14. The air freshener of Claim 13 wherein the wick is supported by the container, and wherein the wick is in contact with the carrier.

15. The air freshener of Claim 13 or 14 further comprising a solvent, at least a portion of the solvent being supported within the container.

16. The air freshener of any of Claims 13-15 wherein the encapsulation material comprises a polysaccharide and/or a polyhydroxy compound.

17. The air freshener of any of Claims 14-16 wherein the encapsulation material is water soluble

and the solvent is water.

18. The air freshener of any of Claims 13-17 wherein the encapsulation material consists essentially of a water-soluble cellular matrix of hydrolyzed starch and at least one other polyhydroxy compound and having globules of fragrance oil in the cells thereof, the said other polyhydroxy compound being at least 20% by weight of the cellular matrix and being a water-soluble member selected from alcohols, plant-type sugars, lactones, monoethers and acetals.

19. The air freshener of any of Claims 13-18 which comprises water in the container and the wick comprises an upper end and a lower end, the lower end being positioned in contact with the water and the upper end being positioned in contact with the carrier.

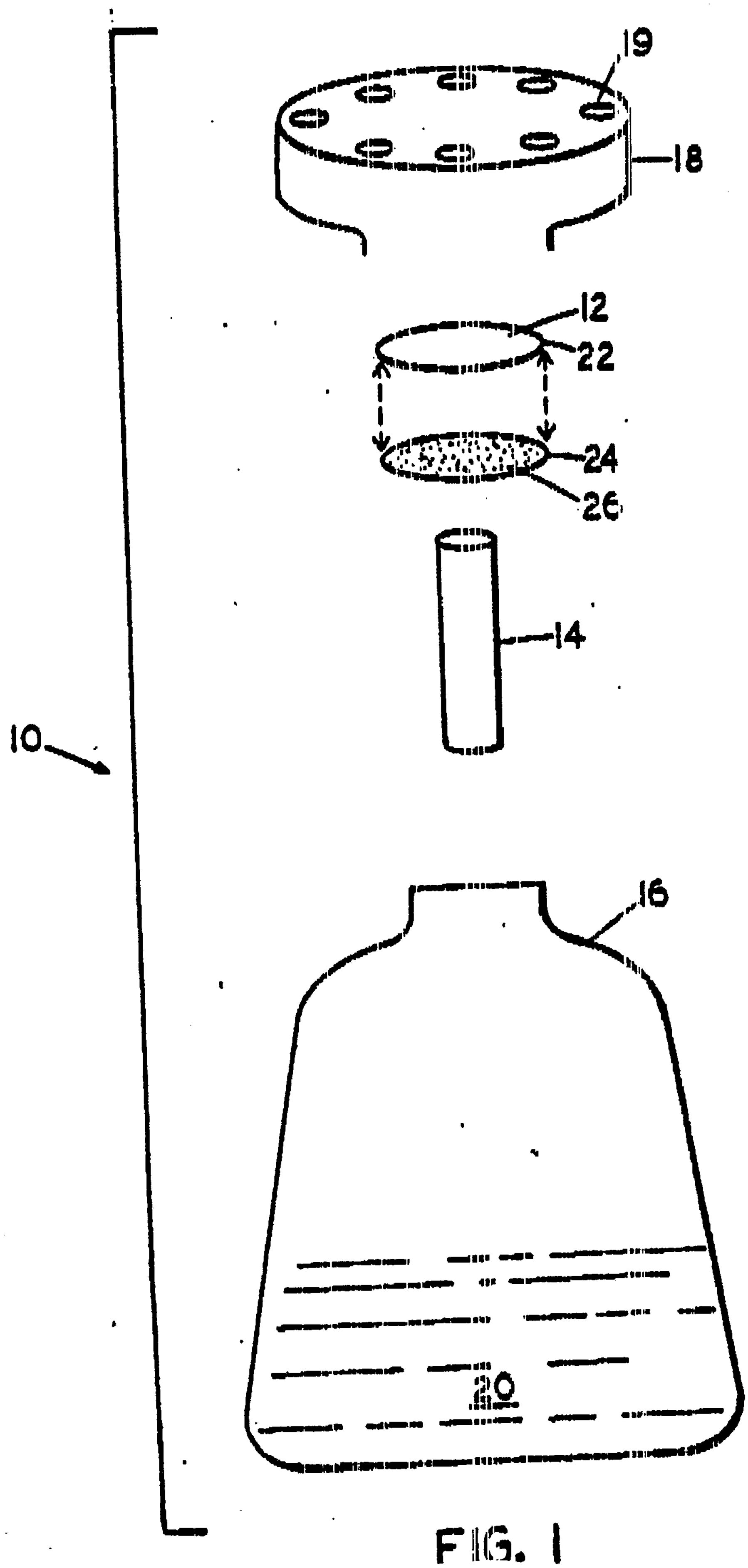


FIG. 1

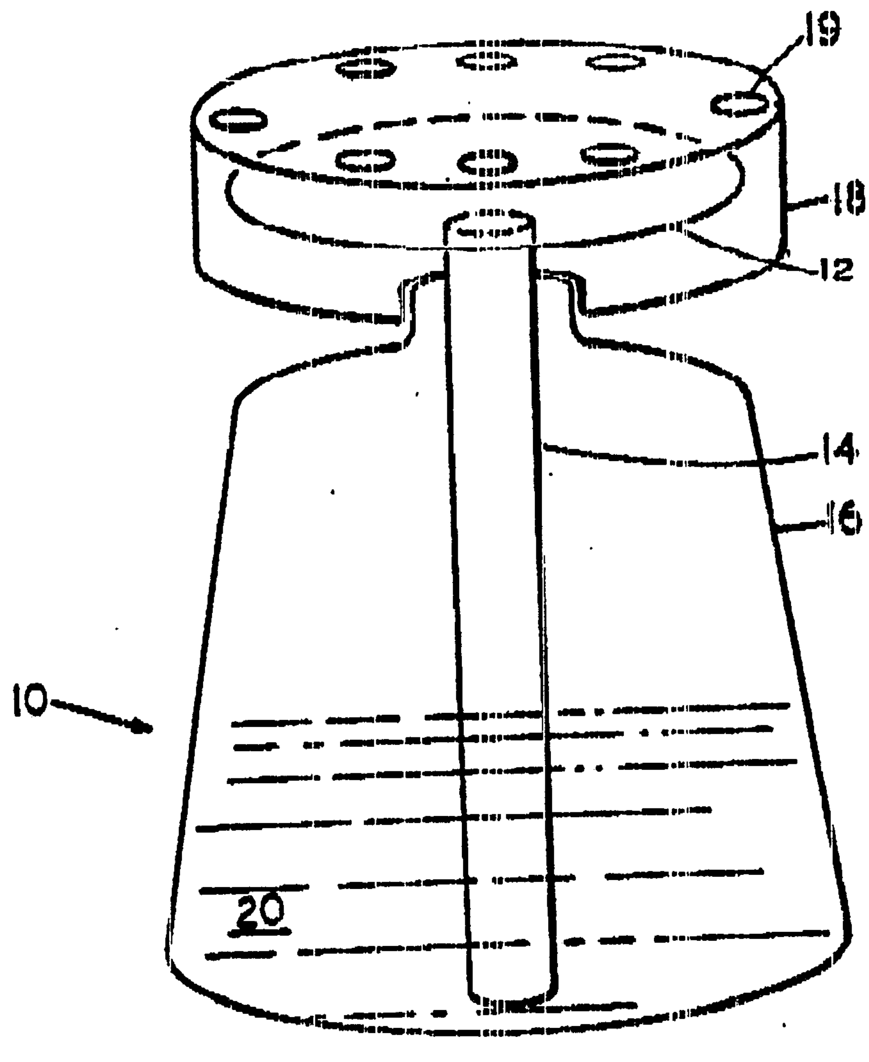


FIG. 2